(19) Europäisches Patentamt
European Patent Office
Office européen des brevets

(11) EP 2 205 582 B1

(12) **EUROPEAN PATENT SPECIFICATION**

(45) Date of publication and mention of the grant of the patent:
**18.07.2012 Bulletin 2012/29**

(21) Application number: **08846258.5**

(22) Date of filing: **06.11.2008**

(51) Int Cl.:
***C07D 335/16*** *(2006.01)*

(86) International application number:
**PCT/GB2008/051033**

(87) International publication number:
**WO 2009/060235 (14.05.2009 Gazette 2009/20)**

(54) **MULTI-FUNCTIONAL THIOXANTHONE PHOTOINITIATORS**
MULTIFUNKTIONALE THIOXANTHON-PHOTOINITIATOREN
PHOTO-INITIATEURS DE TYPE THIOXANTHONES MULTIFONCTIONNELS

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **09.11.2007 GB 0722067**

(43) Date of publication of application:
**14.07.2010 Bulletin 2010/28**

(73) Proprietor: **Lambson Limited**
**Wetherby, Yorkshire LS23 7BJ (GB)**

(72) Inventors:
- **ANDERSON, David George**
  **York, Yorkshire YO30 1XZ (GB)**
- **HARPER, Kevin**
  **Pontefract, Yorkshire WF8 2PG (GB)**
- **ROSE, Alan Thomas**
  **York, Yorkshire YO24 1LT (GB)**

(74) Representative: **Brierley, Anthony Paul et al**
**Appleyard Lees**
**15 Clare Road**
**Halifax**
**HX1 2HY (GB)**

(56) References cited:
**EP-A- 1 380 580**
**WO-A-97/49664**
**WO-A-03/033492**
**CN-A- 1 594 369**
**ES-A6- 2 015 341**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 205 582 B1

## Description

This invention relates to photoinitiators and provides compounds per se, a method of making such compounds, a product and composition comprising such compounds and a method of forming a cured coating on a substrate.

Photoinitiators used in energy curable surface coating formulations are well known and need to possess a range of properties. Such photoinitiators are disclosed in WO 03/33492. For example, they should have good curing activity, be readily soluble in resin formulations and not significantly affect the viscosity or other properties of the formulations and they should be relatively easy to make. One additional important requirement is that there should be a low, or minimal, tendency of such photoinitiators to migrate out of cured coatings since this may taint products in contact with coatings and be a health hazard.

It is an object of the invention to address problems associated with photoinitiators.

The invention is based on the discovery of compounds which can be readily manufactured, are generally in a form (e.g. solid) which can easily be incorporated into formulations comprising curable resins, with little viscosity change of such resin formulations and, importantly, such photoinitiators have a very low tendency to migrate from cured films.

According to a first aspect of the invention, there is provided a photoinitiator compound as described in claim 1.

Unless otherwise stated herein any alkyl, alkenyl, alkylyl or alkenylyl moiety preferably includes carbon and hydrogen atoms only.

X may be selected from fluorine, chlorine, bromine and iodine atoms. X preferably represents a chlorine atom.

Y may represent a $_{C1-12}$, preferably a $C_{1-10}$, more preferably a $C_{1-6}$ alkyl or alkenyl moiety.

Preferably, Y represents a moiety $-(CH)_nR^3-$ wherein n is a number from 1 to 6 and $R^3$ represents a hydrogen atom or an unsubstituted methyl or ethyl group. n is preferably in the range 1 to 3.

Preferably, Y represents a $-CH_2-$ moiety. Preferably, each y represents a $-CH_2-$ moiety.

Preferably, y is a number from 3 to 10. Still more preferably, y is a number from 3 to 6.

Preferably, $A^1$ includes a moiety $-O[CH(CH_3)CH_2O]_2-$ wherein z is an integer.

Preferably, the compound of formula I includes on average 5 to 30, more preferably 5 to 20, especially 7 to 11 moieties of formula $-O[CH(CH_3)CH_2)]_y-$.

Q preferably includes a -O- moiety bonded to moiety $A^1$ in the compound of formula I. A $-O-CH_2-$ moiety is preferably bonded to moiety $A^1$ in the compound of formula I via its -O- atom.

Preferably x moieties bonded to Q by moiety $A^1$ are bonded to Q by -O- moieties which form part of Q. Each -O-moiety of Q may be directly bonded to a $-CH_2$ moiety. Each $-O-CH_2-$ moiety of Q may be bonded to the same carbon atom.

Preferably, Q is a residue of a polyhydroxy compound having 2 to 6, preferably 3 to 5, especially 4 hydroxy groups.

Q may be a residue of ethylene glycol, propylene glycol, butylene glycol, glycerol, trimethylolpropane, ditrimethylolpropane, pentaerythritol or di-pentaerythritol.

Preferably, Q is a reside of a pentaerithritol.

The moiety $Q-(A^1)_x-$ of the compound of formula I preferably has a molecular weight (g/mol) of less than 2000, preferably less than 1500, more preferably less than 1000, especially less than 800. Said molecular weight may be at least 200, preferably at least 400, more preferably at least 600.

Preferably, x is no greater than the number of hydroxy groups in the polyhydroxy compound of which Q is a residue.

Preferably, x is no greater than 4.

Said moiety $-YCO-A^1-$ in the compound of formula I is preferably bonded to the thioxanthone moiety at a para position relative to the position of X.

Preferably, a plurality of moieties (preferably each moiety) bonded to the linking atom or group Q is the same.

Suitably, said compound of formula I has a melting point of at least 50°C, preferably at least 60°C, more preferably at least 70°C. The melting point may be less than 200°C, or less than 100°C.

According to a second aspect of the invention, there is provided a method of making a photoinitiator compound of formula I of the first aspect, the method comprising:

(i) selecting an isolated salt of a compound comprising an anion of formula

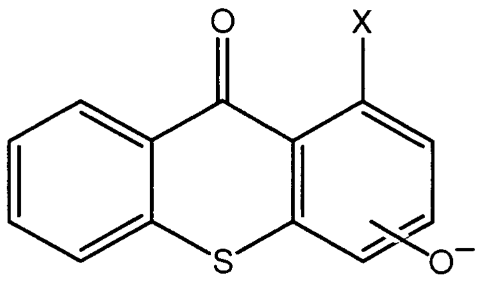

IV

(ii) treating said isolated salt with one or more reagents to produce said compound of formula I.

The method preferably includes a step of preparing a compound of formula IV and isolating a salt thereof which is preferably substantially pure.

The method preferably includes a step of forming an ester of the compound of formula IV. For example, the ester may be of formula

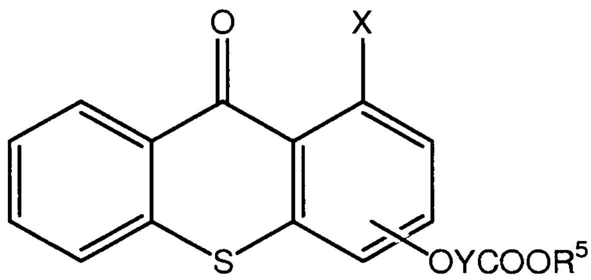

V

wherein $R^5$ is an alkyl group, especially an ethyl group.

X and Y in said moieties of formulae IV and V may be as described in accordance with the first aspect.

The method may involve treatment with a polyhydroxy compound which is arranged, after reaction, to define said moiety $-A^1Q$. A preferred polyhydroxy compound is a propoxylated pentaerithritol.

The method may include comminuting a solid material produced in the process.

According to a third aspect of the invention, there is provided a product comprising or consisting of a compound according to the first aspect in powderous form.

According to a fourth aspect of the invention, there is provide a formulation comprising a compound according to the first aspect and at least one other material selected from: a polymerisable material;
another photoinitiator, which is different to said compound of formula I;
an amine synergist.

Said polymerisable material may comprise a monomer which is ethylenically unsaturated, such as an acrylate or a cationic monomer (e.g. epoxy or vinyl ether).

The formulation of the fourth aspect may be in liquid form. It preferably includes a said polymerisable material. The compound of formula I may be dissolved or dispersed (preferably dissolved) in the polymerisable material.

According to a fifth aspect of the invention, there is provided a method of forming a cured coating on a substrate, the method comprising contacting the substrate with an uncured formulation arranged to provide said coating, wherein said uncured formulation includes a compound of formula I according to the first aspect and exposing the uncured formulation to radiation, for example UV radiation.

The uncured formulation may be as described according to the fourth aspect.

Said coating may comprise an ink.

Said coating may have an thickness in the range 1 to 500 $\mu$m, preferably 1 to 200 $\mu$m.

Any feature of any aspect of any invention or embodiment described herein may be combined with any feature of any aspect of any other invention or embodiment described herein *mutatis mutandis.*

The invention will now be described, by way of example, with reference to the following figures, wherein:

Figure 1 shows the UV spectrum of a photoinitiator of a preferred embodiment of the invention;

Figure 2 shows the UV spectra of films extracted with acetonitrile;

Figure 3 details certain materials referred to herein.

The following materials are referred to hereinafter:

Surfac N090 - a nonyl phenol condensate with nominally 9 moles of ethylene oxide; obtained from Surfachem Group, UK.
Perstorp PS4360 - a propoxylated pentaerithritol having a degree of propoxylation of about 8.5
Vertec TIPT - a titanium alkoxide, tetra-iso-propyl titanate obtained from Johnson Matthey, UK.
Chemcarb GCX - a wood-based carbon obtained from CPL Carbon Link, UK.
Speedcure 7005 - see Figure 3(a)
Speedcure 7040 - see Figure 3(b)

In the description which follows examples 1 and 2 relate to preparation of a photoinitiator of a preferred embodiment of the invention; and thereafter the results of relevant tests undertaken on materials are described.

<u>Example 1</u> - Preparation of polymeric thioxanthone photoinitiator (referred to herein as "thioxanthone photoinitiator A") - first method.

<u>Step 1</u> - <u>Preparation of 1-chloro-4-hydroxythioxanthone</u>

To a 250ml round-bottomed flask was charged 98% sulphuric acid (275.4g) and to this water (6.1g) was added to reduce the acid strength to 96%. To this was added dithiobisbenzoic acid (30.6g; 0.10g mole) and the mixture stirred for 30 minutes. p-Chlorophenol (58.1g; 0.45g mole) was then added gradually over 1.5 hours, the temperature being maintained at 40-45°C. A 60-minute workout at this temperature yielded a mobile orange/red slurry. This was warmed slowly to 80-85°C and held for 2 hours, steady sulphur dioxide evolution occurring during this period. The mixture was finally warmed to 100°C and held for 30 minutes before then cooling the resultant deep red solution. This solution was poured slowly into water (269ml) at 50°C, allowing the temperature to rise to 88°C. After self-cooling to 50°C the mixture was filtered and the filter cake washed three times with water (100ml). A buff coloured solid was obtained that was then oven-dried to constant weight.

Weight obtained = 40.9g (77.9% yield from DTBBA)

<u>Step 2</u> - <u>Preparation of 1-chloro-4-hydroxythioxanthone, sodium salt</u>

1-chloro-4-hydroxythioxanthone (52.5g; 0.20 g mole) prepared in step 1 was slurried in water (328ml) and Surfac NO90 (0.3g) added. This was heated to 80-85°C and then basified by adding 10% caustic soda solution (90g; 0.225 g mole) over 30 minutes. The mixture was cooled to 5°C over 1.5 hours and held at this temperature for 30 minutes. The resulting slurry was filtered, the cake washed with cold water (80ml) and the sodium salt then dried to yield 36.4g.

Using the combined mother and wash liquors a second portion of 1-chloro-4-hydroxythioxanthone (52.5g) was converted to sodium salt as above, but without the addition of extra surfactant. After drying 53.4g was obtained.

Combined yield from these two cycles was 89.8g (78.9% from 1-chloro-4-hydroxythioxanthone)

<u>Step 3</u> - <u>Preparation of 1-chloro-4-carboxymethyoxythioxanthone, ethyl ester</u>

1-chloro-4-hydroxythioxanthone, sodium salt (142.2g; 0.500g mole) of step 2, methylethylketone (1000ml), dimethylsulphoxide (10g) and ethylbromoacetate (96.0g; 0.575 g mole) were charged to a 2-litre flask and heated to reflux (80°C). The initial red/orange colour of the slurry changed to a beige/brown as the reaction proceeded. After 3 hours at reflux extra methylethylketone (380ml) was added. The course of the reaction was followed by TLC. This involved placing a few drops of the mixture in a 7ml vial, adding a small volume of water and 1N HCl to acidify. Sufficient acetone was then added to give a clear solution. The solution was spotted on a silica gel plate containing fluorescent indicator (254nm) and run in 1:1 acetone/petroleum ether. Viewed under a fluorescent lamp the product was seen to have an RF of approx. 0.51 and the chlorohydroxythioxanthone starting material an RF of 0.47. After 4.5 hours at reflux the reaction was complete.

The mixture was cooled to ambient temperature and sodium methoxide powder (4.04g; 0.075 g mole) added to react with any remaining ethylbromoacetate. After stirring for 1 hour the mixture was warmed to 70°C and held for a further 30 minutes. It was then filtered through a pre-warmed Buchner funnel to remove the by-product sodium bromide,

the cake being washed with hot methylethylketone (30ml). Approximately 52g of insolubles were collected.

The filtrate was then cooled over about 2 hours to 0-5°C and held for a further 30 minutes before filtering through a Buchner funnel to collect the precipitated product. The cake was given a small displacement wash with cold methylethylketone (30ml) and the product then dried at 70°C. The dried material was a yellowish khaki colour. First crop yield was 127.2g (73.0% from CHTX Na salt)

The volume of filtrate from product isolation was 1300ml. Using a rotary evaporator solvent was removed to reduce the volume to 300ml and this was then cooled to 0-5°C and held for 30 minutes. Filtration and drying of the cake collected gave a second crop of product, identical in appearance to the first crop.

Second crop yield was 16.2g (9.3% from CHTX Na salt)

On TLC in 1:1 acetone/petroleum ether both crops of material exhibit only one spot.

Overall yield was 143.4g (82.3% from CHTX Na salt)

Step 4 - Preparation of polymeric thioxathone photoinitiator (thioxanthone photoinitiator A)

The ethyl ester of Step 2 was transesterified with Perstorp PS4360 as represented and described below.

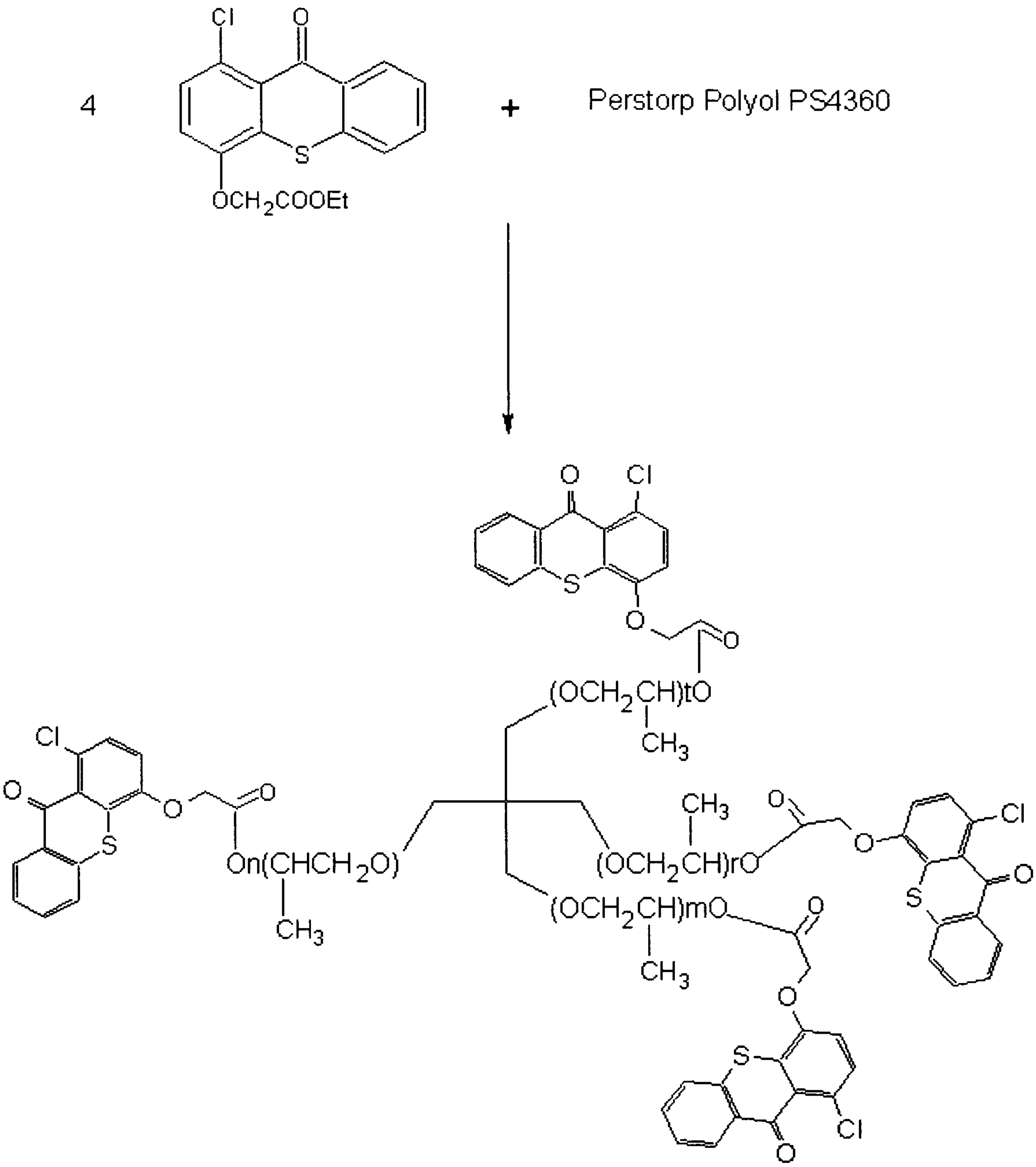


Thioxanthone photoinitiator A

A 1-litre flask was charged with PS4360 (94.4g; 0.15 mole), CHTX-ethyl ester (198.6g; 0.57 moles) and toluene (150mls). Any entrained water was azeotroped out of the system using a Dean and Stark apparatus. After cooling, the Dean and Stark apparatus was replaced by a 150mm vacuum jacketed Vigreux column, and still head, and the system was thoroughly purged with nitrogen. Vertec TIPT (0.5g) transesterification catalyst was added, and the reaction heated to steady reflux. The still base temperature was about 120°C initially, the still head temperature was 79°C, indicating that ethanol was forming as expected. The ethanol was taken off slowly, such that a steady reflux was maintained, and the still head remained at 78°-79°C. Taking ethanol off too quickly causes the still head to rise as toluene is leached from the system. The reaction was monitored by TLC (1:2 acetone : petroleum ether 100-120). During the last 5 hours of reaction, the head temperature intermittently reached 110°C. When this occurred, a further 0.5g TIPT was added, and the column was intermittently placed on total reflux until remaining ethanol formation brought the head temperature back down to 78°C, then take-off was resumed (i.e. intermittent take-off was used at the end of the reaction to ensure that most of the toluene stayed in the system). However, after 18 Hrs, the rate of take-off was increased so that some

of the toluene was removed, allowing the base temperature to increase and drive the reaction to completion. After 24 Hrs, 95.8g distillate had been removed and the final base temperature was 140°C. A total reaction time of 24Hrs gave complete reaction as monitored by TLC.

After cooling, acetone (500mls) was added, together with sodium dihydrogen phosphate (3g in 3g water), and the reaction mixture was refluxed for 30 minutes. This facilitated deactivation/removal of TITP catalyst. After cooling to 40°C, anhydrous magnesium sulphate (6g) and Chemcarb GCX (2g) was added, and reflux continued for 1.5 Hrs, to dry and decolourise the solution. After cooling to 40°C, the inorganics were filtered via a celite bed, and washed with acetone (100mls). The acetone was removed initially at atmospheric pressure and then at up 95°C and 15mm Hg for 2 Hrs. The time taken was that required to ensure no solvent remained in the product melt, as signified by cessation of its bubbling due to solvent evaporation.

The yield was 264.8g of pale orange viscous oil (96.0% theory).

Finally the product melt was poured onto a glass Petri dish, and allowed to set into a pale orange glass. This was removed, and powdered readily using a mortar and pestle, to give a pale yellow free-flowing solid.

The product had a melting point of 78-80°C and an overall yield, based on the amount of 1-chloro-4-hydroxthioanthone selected in step 1, of 64%.

<u>Example 2</u> - Preparation of polymeric thioxanthone photoinitiator A - second method.

This method is based on the method described in US6960668B and is used to produce the same photoinitiator as that prepared in Example 1 and represented as the product of the reaction of Example 1, step 4.

<u>Step 1</u> - <u>Preparation of Thioxanthone</u>

4-chlorophenoxyacetic acid was condensed with dithiobisbenzoic acid to yield the carboxymethoxy intermediate as the acid which was precipitated in water and isolated in a first filtration. This was then purified, in a second filtration, from a hot mixed solvent system before being used in the next stage of the process. The first filtration was reasonably quick but after the reslurry in the mixed solvent system it was extremely slow and a very low solids content cake was obtained. Consequently, a high proportion of materials in the liquor is dried back onto the filter cake. The yield at this point was 99.5%.

<u>Step 2</u> - <u>Preparation of polymeric thioxanthone photoinitiator A</u>

The carboxylic acid intermediate prepared in Step 1 was esterified with the required polyol in toluene, using PTSA as catalyst. A solution of the product was obtained which was washed with dilute base and then water before the product was removed under vacuum to leave the product as a glassy solid. A yield of 61% was obtained. The acid intermediate is of low solubility in toluene (unlike the ester of Example 1 that dissolves readily on warming) and consequently an extended reaction time was needed to complete the esterification - in the region of 45 hours. Due to the presence of interfacial material the separations from the aqueous washes are slow.

Although the product was obtained as a glass that breaks up reasonably easily it tends to cake and would thus present handling problems. This indicates a slightly lower purity than from the Example 1 route.
Overall yield was 60.7%, the products melting point could not be determined as it began to soften at ~ 25°C.

<u>Comparison of Examples 1 and 2</u>

The Example 1 method appears to produce an advantageous product. It is preferred that the product is of sufficient quality that it may be ground, subsequently remain as a powder and show no signs of caking that would make handling problematic. This is the case for the Example 1 material but not for the example 2 material.

The good quality of the final product obtained is a culmination of the previous steps wherein the presence of impurities carrying forward is minimised by isolation of the intermediates. Although the isolations incur extra processing time and some yield loss they ensure that the final product is of the required quality.

**Assessment of materials**

Assessment 1 - Viscosity

The viscosity of a formulation comprising a typical resin mixture comprising Bisphenol A EO-2 (70%) and TPGDA (30%) (an acrylic acid ester) was assessed using a Brookified viscometer, under conditions of Spindle 2, 60rpm, 25°C. The viscosity of the same resin mixture containing, additionally, polymeric thioxanthone photoinitiator A (2%) was

assessed in the same way. Results are tabulated below.

| Formulation | Viscosity (cp) |
|---|---|
| resin mixture | 190 |
| resin mixture + polymeric thioxanthone photoinitiator A | 200 |

It will be noted that, advantageously, addition of the polymeric thioxanthone photoinitiator A affects the viscosity of the formulation to only a relatively small extent.

Assessment 2 - UV spectrum

The UV spectrum of the thioxanthone photoinitiator A was assess and is shown in figure 1. From the spectrum it is clear that thioxanthone photoinitiator A absorbs in appropriate regions so that it may be irradiated with an appropriate UV irradiation source.

Assessment 3 - cure testing

A formulation comprising polymeric photoinitiators(including thioxanthone photoinitiator A) was compared to a formulation comprising equivalent monomeric photoinitiators in cure tests.

Formulations were made up in an epoxy acrylate resin comprising of 70 EO-2 bisphenol A diacrylate, 30% TPGDA and as near an equivalent molar amount of the various types of photoinitiators subjected to testing. The testing programme was carried out using a Dymac Hi5 belt curing machine equipped with a 900w mercury diffusion lamp. The test samples were cured in air for the number of passes displayed in the table below. Degree of cure was assessed by simple touch test for initial cure and thumb twist test for through cure. Belt speed in all cases was 16.5 m/min.

| Formulation No. | parts per hundred resin | Speedcure CPTX | Speedcure BP | Speedcure EDB | Speedcure 7005 | Thioxanthone photoinitiator A | Speedcure 7040 |
|---|---|---|---|---|---|---|---|
| 1 | 100 | 2 | 2 | 4 | | | |
| 2 | 100 | | | | 4.3 | 2.0 | 7.7 |

Results of the tests were as follows:

| Formulation No. | Touch dry passes 4 μm | Thumb twist passes 4 μm | Touch dry passes 2 μm | Thumb twist passes 2 μm |
|---|---|---|---|---|
| 1 | 4 | 11 | 3 | 6 |
| 2 | 3 | 7 | 3 | 4 |

It will be appreciated from the results that a speed of cure of the polymeric photoinitiators comparable with equivalent monomeric photoinitiators can be achieved.

Assessment 4 - Migration test 1

A film comprising formulation no. 2 of Assessment 3 was cured by doubling the number of passes taken to achieve a pass on the thumb twist test. A migration test was then carried out by subjecting the film to a solution of 90% ethanol/10% water for 2 hours at 60°C. The leachate was analysed by HPLC to determine the amount of migration of the photoinitiators. Results are tabulated below wherein migration is expressed as % of each material present in the cured formulation which has migrated.

| Photoinitiator component | Migration % |
|---|---|
| Speedcure 7040 | 7.5 |
| Speedcure 7005 | 6.8 |
| Thioxanthone photoinitiator A | 0 |

Assessment 5 - Migration test 2

Following a process similar to that described in Assessment 4, cured 4 μm films of formulations 1 and 2 of Assessment 3 were extracted with acetonitrile. UV spectra for the acetonitrile extracts are provided in figure 2. It will be noted that, for the formulation 2, there is no absorbance at 380nm (compare figure 1) indicating that none of the thioxanthone photoinitiator A has been extracted into the acetonitrile.

Conclusion

It will be noted that thioxanthone photoinitiator A is readily handled and incorporated into resin formulation due to it being solid and, when introduced, it has a minimal effect on viscosity. The activity of the thioxanthone photoinitiator A is comparable to that of equivalent monomeric photoinitiators. Particularly advantageously, it is found that thioxanthone photoinitiator A exhibits negligible migration.

## Claims

1. A photoinitiator compound of formula

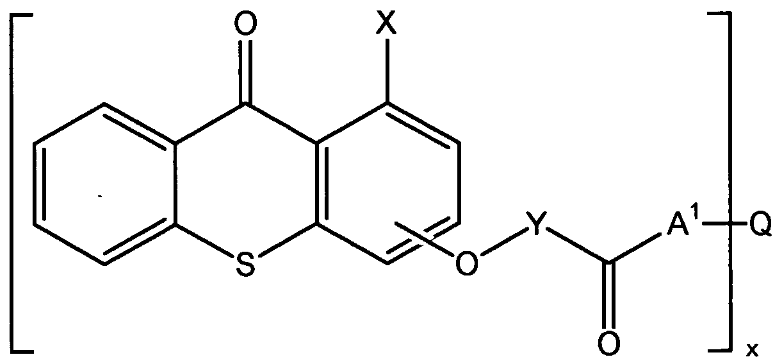

I

wherein:

X represents a halogen atom;
Y represents an alkylyl or alkenylyl moiety;
$A^1$ is a group of formula - $[OCH_2CH_2]_y$-, -$[OCH_2CH_2CH_2CH_2]_y$- or -$[OCH(CH_3)CH_2]_y$-, where y is a number from 1 to 10, or a group of formula -$[O(CH_2)_bCO]_y$- or - $[O(CH_2)_bCO]_{(y-1)}$-$[O(CHR^2CHR^1)_a]$-, where b is a number from 4 to 5 and y is a number from 1 to 10;
Q is a residue of a polyhydroxy compound; and
x represents a number greater than 1, wherein moieties bonded to group Q may be the same or different to one another.

2. A compound according to claim 1, wherein X represents a chlorine atom.

3. A compound according to claim 1 or claim 2, wherein Y represents a moiety -$(CH)_nR^3$- wherein n is a number from 1 to 6 and $R^3$ represents a hydrogen atom or an unsubstituted methyl or ethyl group.

4. A compound according to any preceding claim, wherein Y represents a -$CH_2$- moiety.

5. A compound according to any preceding claim, wherein $A^1$ includes a moiety-$[OCH(CH_3)CH_2]_y$-, wherein y is a number from 1 to 10.

6. A compound according to any preceding claim, wherein Q is a residue of ethylene glycol, propylene glycol, butylene glycol, glycerol, trimethylolpropane, ditrimethylolpropane, pentaerythritol or di-pentaerythritol.

7. A compound according to any preceding claim, wherein the moiety Q-$(A^1)_x$- of the compound of formula I has a molecular weight (g/mol) of less than 2000.

**8.** A compound according to any preceding claim, wherein said moiety -YCO-$A^1$- in the compound of formula I is bonded to the thioxanthone moiety at a para position relative to the position of X.

**9.** A method of making a photoinitiator compound of formula I of any preceding claim, the method comprising:

(i) selecting an isolated salt of a compound comprising an anion of formula

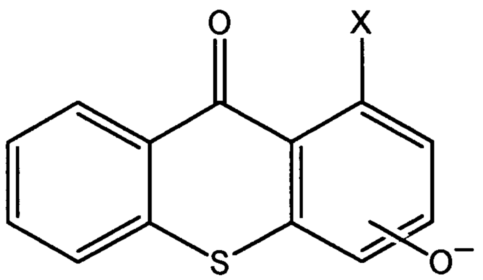

IV

(ii) treating said isolated salt with one or more reagents to produce said compound of formula I.

**10.** A formulation comprising a compound according to any of claims 1 to 8 and at least one other material selected from: a polymerisable material;
another photoinitiator, which is different to said compound of formula I; and
an amine synergist.

**11.** A method of forming a cured coating on a substrate, the method comprising contacting the substrate with an uncured formulation arranged to provide said coating, wherein said uncured formulation includes a compound of formula I according to any of claims 1 to 8, and exposing the uncured formulation to radiation.

**Patentansprüche**

**1.** Photoinitiatorverbindung der Formel

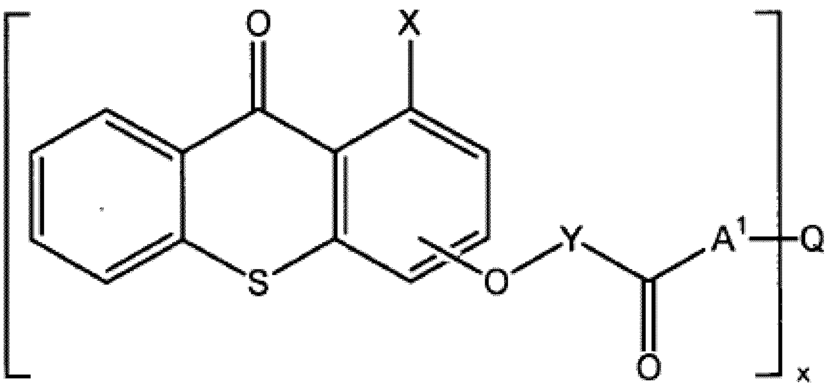

I

worin:

X für ein Halogenatom steht;
Y für eine Alkylyl- oder Alkenylylgruppierung steht;
$A^1$ für eine Gruppe der Formel $-[OCH_2CH_2]_y-$, $-[OCH_2CH2CH_2CH_2]_y-$ oder $-[OCH(CH_3)CH_2]_y-$, wobei y für eine Zahl von 1 bis 10 steht, oder eine Gruppe der Formel $-[O(CH_2)_bCO]_y-$ oder $-[O(CH_2)_bCO]_{(y-1)}-[OCHR^2CHR^1)_a]-$, wobei b für eine Zahl von 4 bis 5 steht und y für eine Zahl von 1 bis 10 steht, steht;
Q für einen Rest einer Polyhydroxyverbindung steht und
x für eine Zahl größer 1 steht, wobei an die Gruppe Q gebundene Gruppierungen gleich oder voneinander verschieden sein können.

**2.** Verbindung nach Anspruch 1, wobei X für ein Chloratom steht.

**3.** Verbindung nach Anspruch 1 oder Anspruch 2, wobei Y für eine Gruppierung $-(CH)_nR^3-$ steht, wobei n für eine Zahl von 1 bis 6 steht und $R^3$ für ein Wasserstoffatom oder eine unsubstituierte Methyl- oder Ethylgruppe steht.

**4.** Verbindung nach einem der vorhergehenden Ansprüche, wobei Y für eine $-CH_2$-Gruppierung steht.

**5.** Verbindung nach einem der vorhergehenden Ansprüche, wobei $A^1$ eine Gruppierung $-[OCH(CH_3)CH_2]_y-$, wobei y für eine Zahl von 1 bis 10 steht, einschließt.

**6.** Verbindung nach einem der vorhergehenden Ansprüche, wobei Q für einen Rest von Ethylenglykol, Propylenglykol, Butylenglykol, Glycerin, Trimethylolpropan, Ditrimethylolpropan, Pentaerythrit oder Dipentaerythrit steht.

**7.** Verbindung nach einem der vorhergehenden Ansprüche, wobei die Gruppierung $Q-(A^1)_x$ der Verbindung der Formel I ein Molekulargewicht (g/mol) von weniger als 2000 aufweist.

**8.** Verbindung nach einem der vorhergehenden Ansprüche, wobei die Gruppierung $-YCO-A^1-$ in der Verbindung der Formel I in einer para-Position relativ zur Position von X an die Thioxanthongruppierung gebunden ist.

**9.** Verfahren zur Herstellung einer Photoinitiatorverbindung der Formel I nach einem der vorhergehenden Ansprüche, bei dem man:

(i) ein isoliertes Salz einer Verbindung mit einem Anion der Formel

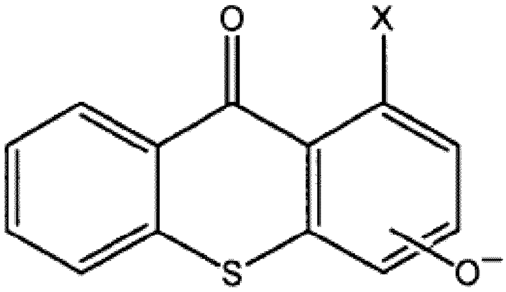

IV

auswählt und
(ii) das isolierte Salz mit einem oder mehreren Reagezien zu der Verbindung der Formel I umsetzt.

**10.** Formulierung, umfassend eine Verbindung nach einem der Ansprüche 1 bis 8 und mindestens eine andere Substanz, ausgewählt aus: einer polymerisierbaren Substanz;
einem anderen Photoinitiator, der von der Verbindung der Formel I verschieden ist; und
einem Amin-Synergisten.

**11.** Verfahren zur Bildung einer gehärteten Beschichtung auf einem Substrat, bei dem man das Substrat mit einer zur Bereitstellung der Beschichtung vorgesehenen ungehärteten Formulierung in Berührung bringt, wobei die ungehärtete Formulierung eine Verbindung der Formel I nach einem der Ansprüche 1 bis 8 enthält, und die ungehärtete Formulierung Strahlung aussetzt.

## Revendications

**1.** Composé photo-initiateur de formule

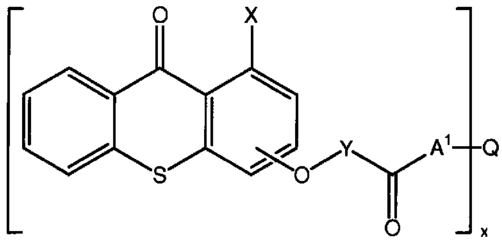

I

dans laquelle
X représente un atome d'halogène ;
Y représente un motif alkylyle ou alcénylyle ;
$A^1$ est un groupement de formule $-[OCH_2CH_2]_y-$, $-[OCH_2CH_2CH_2CH_2]_y-$ ou $-[OCH(CH_3)CH_2]_y-$, où y est un nombre allant de 1 à 10, ou un groupement de formule $-[O(CH_2)_bCO]_y-$ ou $-[O(CH_2)_bCO]_{(y-1)}-[O(CHR^2CHR^1)_a]-$, où b est un nombre allant de 4 à 5 et y est un nombre allant de 1 à 10 ;
Q est un reste d'un composé polyhydroxylé ; et
X représente un nombre supérieur à 1, où les motifs liés au groupement Q peuvent être identiques ou différents les uns des autres.

**2.** Composé selon la revendication 1, dans lequel X représente un atome de chlore.

**3.** Composé selon la revendication 1 ou la revendication 2, dans lequel Y représente un motif- $(CH)_nR^3$-, où n est un nombre allant de 1 à 6 et $R^3$ représente un atome d'hydrogène ou un groupement méthyle ou éthyle non substitué.

**4.** Composé selon l'une quelconque des revendications précédentes, dans lequel Y représente un motif $-CH_2-$.

**5.** Composé selon l'une quelconque des revendications précédentes, dans lequel $A^1$ comporte un motif - $[OCH(CH_3)CH_2]_y-$, où y est un nombre allant de 1 à 10.

**6.** Composé selon l'une quelconque des revendications précédentes, dans lequel Q est un reste d'éthylène glycol, de propylène glycol, de butylène glycol, de glycérol, de triméthylolpropane, de ditriméthylolpropane, de pentaérythritol ou de dipentaérythritol.

**7.** Composé selon l'une quelconque des revendications précédentes, dans lequel le motif $Q-(A^1)_x-$ du composé de formule I possède un poids moléculaire (g/mol) inférieur à 2000.

**8.** Composé selon l'une quelconque des revendications précédentes, dans lequel ledit motif $-YCO-A^1-$ dans le composé de formule I est lié au motif thioxanthone au niveau d'une position para par rapport à la position de X.

**9.** Méthode de préparation d'un composé photo-initiateur de formule I selon l'une quelconque des revendications précédentes, la méthode comprenant :

(i) la sélection d'un sel isolé d'un composé comprenant un anion de formule

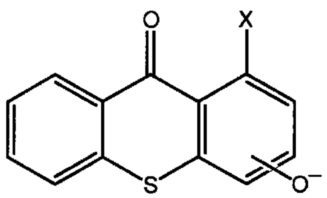

IV

(ii) le traitement dudit sel isolé par un ou plusieurs réactifs, afin de produire ledit composé de formule I.

**10.** Formulation comprenant un composé selon l'une quelconque des revendications 1 à 8, et au moins un autre matériau choisi parmi :

un matériau polymérisable ;
un autre photo-initiateur, qui est différent dudit composé de formule I ; et
un synergiste d'amine.

**11.** Méthode de formation d'un revêtement durci sur un substrat, la méthode comprenant la mise en contact du substrat avec une formulation non durcie disposée afin de fournir ledit revêtement, où ladite formulation non durcie comporte un composé de formule I selon l'une quelconque des revendications 1 à 8, et l'exposition de la formulation non durcie à un rayonnement.

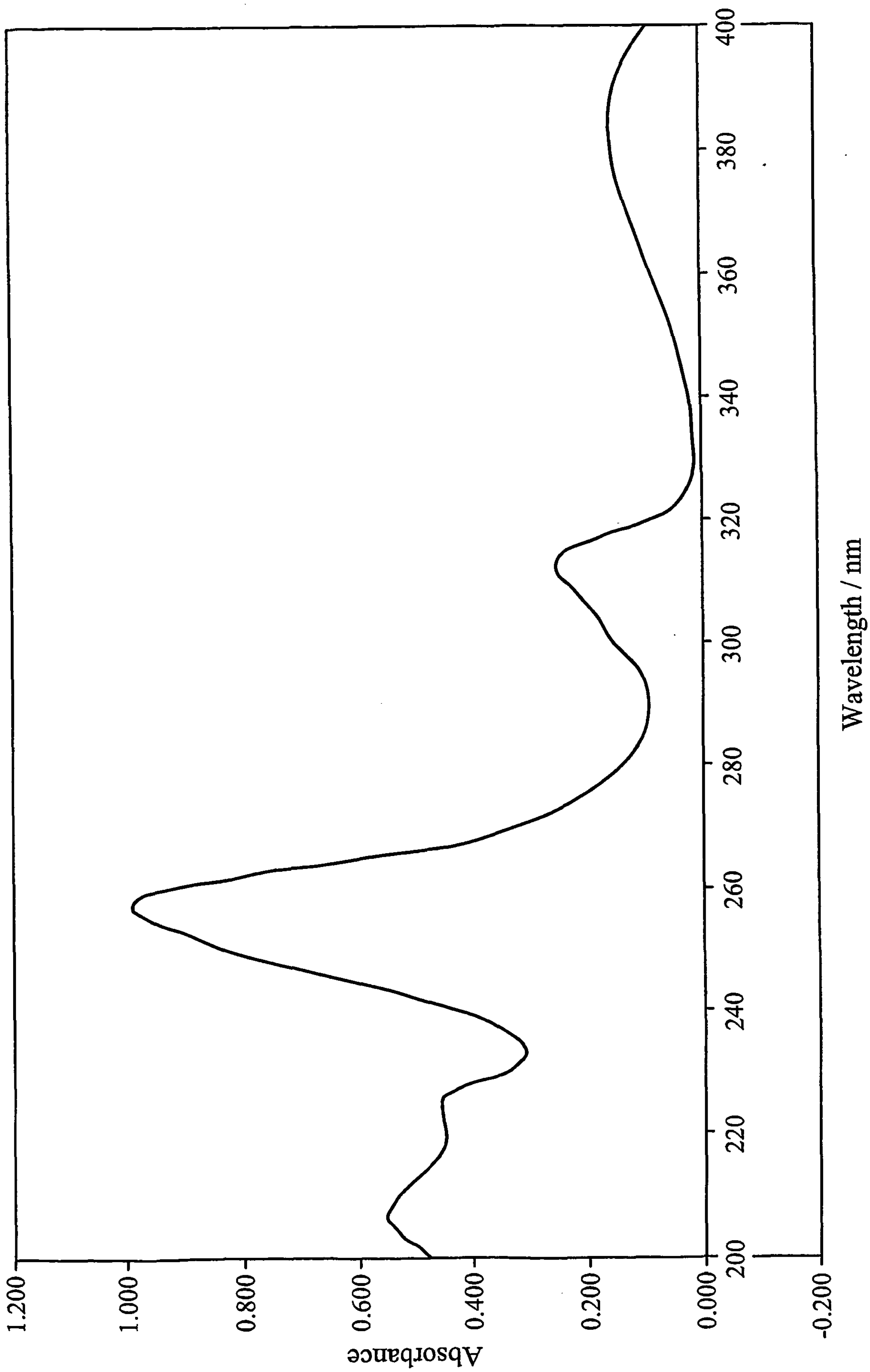
Absorbance
1.200
1.000
0.800
0.600
0.400
0.200
0.000
-0.200
Wavelength / nm
200
220
240
260
280
300
320
340
360
380
400

FIG. 1

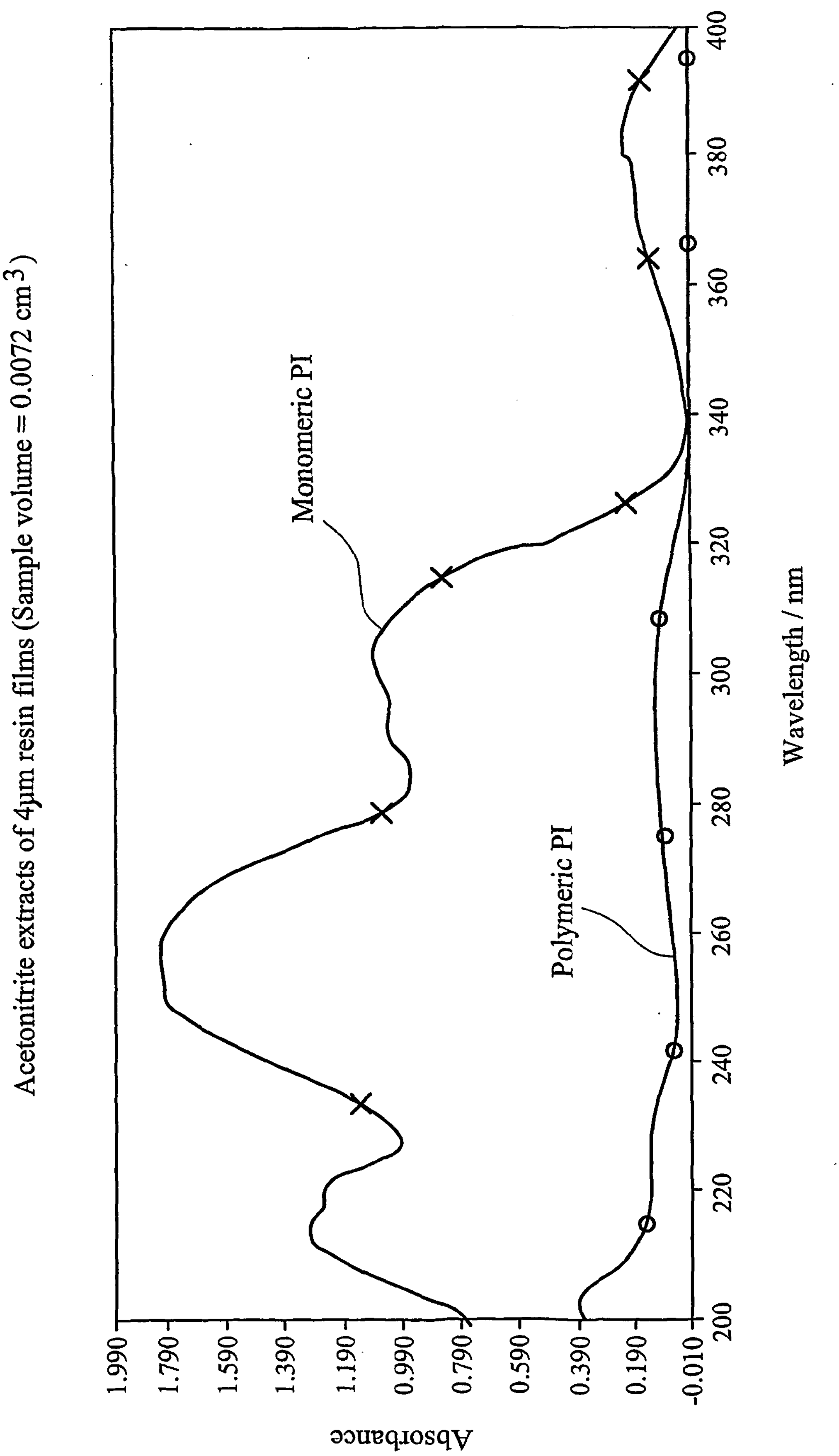
Acetonitrite extracts of 4μm resin films (Sample volume = 0.0072 cm3)
Monomeric PI
Polymeric PI
Absorbance
1.990
1.790
1.590
1.390
1.190
0.990
0.790
0.590
0.390
0.190
-0.010
200
220
240
260
280
300
320
340
360
380
400
Wavelength / nm

FIG. 2

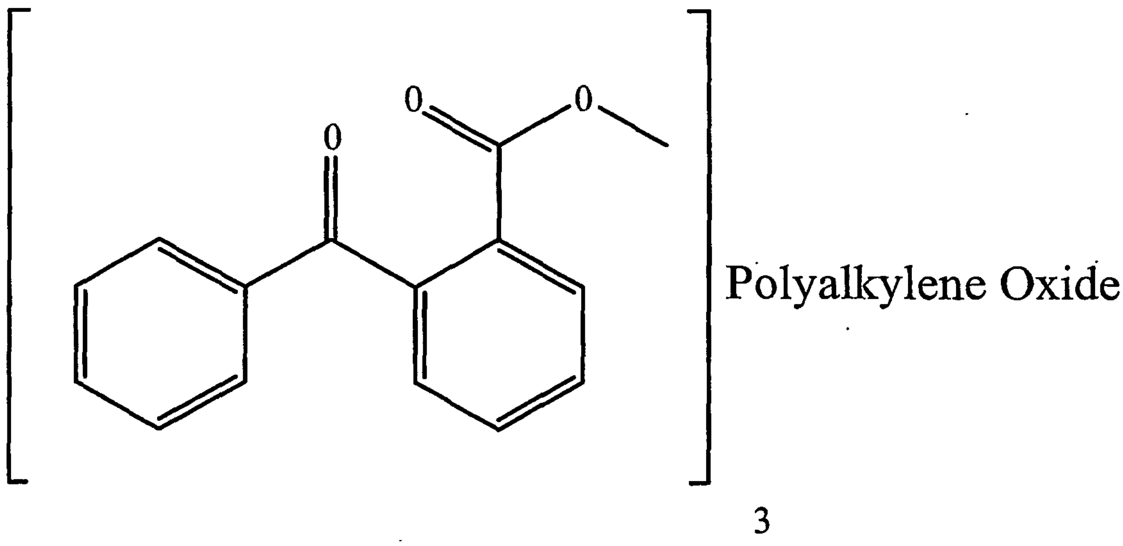

FIG. 3(a)

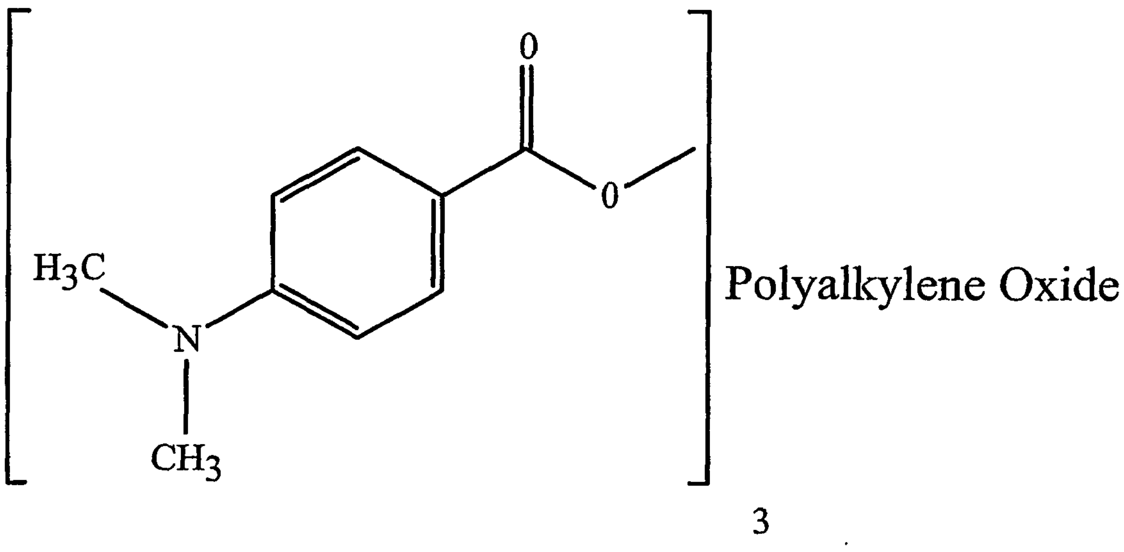

FIG. 3(b)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0333492 A
- US 6960668 B